# FASCICULE DE BREVET EUROPEEN

(11) **EP 1 720 442 B1**
(45) Date de publication et mention de la délivrance du brevet: **22.10.2008**
(21) Numéro de dépôt: 05732918.7
(22) Date de dépôt: 25.02.2005
(51) Int. Cl.: A61B 1/045, A61B 1/24

(54) **CAMERA A USAGE MEDICAL, NOTAMMENT DENTAIRE**
KAMERA FÜR DEN EINSATZ IN DER MEDIZIN, BESONDERS DER ZAHNMEDIZIN
CAMERA FOR MEDICAL, PARTICULARLY DENTAL USE

(30) Priorité: 01.03.2004 FR 0402090
(43) Date de publication de la demande: 15.11.2006
(73) Titulaire: Sopro (Societe Anonyme), 13705 La Ciotat Cedex (FR)
(72) Inventeur: BOYER, Philippe, F-13006 Marseille (FR); MAZUIR, Alain, F-83470 St Maximin la Ste Baume (FR)
(74) Mandataire: Desormiere, Pierre-Louis
(86) Numéro de dépôt international: PCT/FR2005/000456
(87) Numéro de publication internationale: WO 2005/089631

(56) Documents cités:
- US-A- 5 771 067
- US-A1- 2002 049 464

## Description

La présente invention concerne une caméra à usage médical, et notamment dentaire.

On connaît dans le domaine médical, et plus particulièrement dans le domaine dentaire, des micro-caméras dont l'objectif est disposé à l'extrémité d'une pièce à main et qui comportent sur le corps de celles-ci un bouton de commande permettant de déclencher la prise de vue ou de saisir une image spécifique.

Ces micro-caméras, notamment lorsqu'elles sont utilisées dans des applications où il est nécessaire de "geler" une image, sont commandées par une pédale spécifique actionnée par le pied de l'utilisateur. On a constaté en effet que, notamment dans ce type d'applications, le simple actionnement d'un bouton de commande solidaire de la caméra, si doux que soit le déclenchement de celui-ci, avait pour conséquence de générer un mouvement de la caméra ayant pour effet de rendre floue l'image que l'on souhaitait "fixer". Or, notamment dans les cabinets dentaires, on utilise pour de nombreux instruments ce type de commande par pédale, si bien qu'il peut être gênant pour le praticien de multiplier celles-ci.

On connaît par ailleurs différents types de commandes de déclenchement ne nécessitant aucun déplacement physique des doigts de l'utilisateur, tels que par exemple les commandes de type sensitif qui sont constituées notamment par des capteurs capacitifs ou inductifs qui réagissent au champ électrique produit par un doigt de l'utilisateur.

L'une des difficultés rencontrées dans l'utilisation de telles commandes sur des caméras dentaires pour assurer le "gel" d'une image, tient au fait qu'à l'inverse des boutons de commande classiques qui autorisent une localisation tactile, les commandes sensitives ne peuvent être quant à elles localisées que visuellement par le praticien, ce qui n'est pas acceptable en l'espèce dans la mesure où la concentration visuelle de ce dernier doit impérativement s'exercer sur le champ opératoire.

C'est pourquoi on a proposé dans la demande de brevet FR 02 15014, , déposée le 29.11.02 et publiée le 04.06.04 (n'appartient pas à l'état de la technique antérieure selon l'article 54(2) CBE), de prévoir sur la face externe du corps de la caméra une zone de discontinuité de surface, formant un relief ou un creux, qui est disposée au droit du capteur sensitif, et qui permet à l'utilisateur d'assurer le positionnement de son doigt par rapport à la zone de détection légèrement en amont de celle-ci, étant entendu que tout mouvement supplémentaire de ce doigt provoquera sa détection et en conséquence le déclenchement de la commande appropriée.

Les systèmes de commande de type sensitif se composent habituellement d'un capteur (généralement une pastille métallique) et de moyens électroniques disposés sur un circuit support. Or il s'avère que pour des raisons diverses dues notamment à l'encombrement, à la conception globale de la caméra, à l'esthétique du corps de celle-ci, ou à la présence de composants divers sur le circuit support, il advient que, dans certaines configurations, il n'est pas possible de disposer le capteur et son circuit électronique associé en contact avec la face interne du corps de la caméra. Or, on sait qu'il n'est pas envisageable, lorsque l'on souhaite que le capteur détecte de façon précise et répétitive un doigt de l'utilisateur, de séparer le circuit électronique du capteur afin de positionner ce dernier contre la paroi interne du corps à proximité immédiate de la zone de détection. On a en effet constaté que la liaison filaire que l'on établit alors entre ces deux éléments a pour conséquence de détruire la précision et la bonne répétitivité de la position de détection, dans la mesure où les fils de liaison deviennent partie intégrante du capteur, leur position et leur longueur influençant la détection.

Le document US-A-5 771 067 décrit une camera dentaire comportant un boitier allongé pourvu à une extrémité de moyens de prises d'images et comprenant des moyens de commande comportant une zone de détection située sur le boîtier qui est délimitée par une discontinuité de surface.

Le document US-A-2002/0049464 décrit une pièce à main chirurgicale comprenant des moyens de commande de type sensitif. Une configuration écrite pour ces dits moyens consiste en un capteur, compris dans la pièce à main, configuré pour détecter la force des champs induits lors d'une pression sur un élément élastomère comprenant un aimant.

La présente invention a pour but de proposer un moyen permettant de disposer le capteur et son circuit support éloignés de la zone de détection tout en conservant les qualités de précision et de répétitivité que l'on aurait si ce capteur était disposé à proximité immédiate de celle-ci.

La présente invention a ainsi pour objet une caméra de prise de vues médicale, notamment dentaire, comportant un boîtier allongé apte à être tenu par une main d'un utilisateur et pourvu à son extrémité antérieure de moyens de prise d'images, ce boîtier comportant des moyens de commande aptes à "geler" sur des moyens d'affichage une image choisie par l'utilisateur, ces moyens de commande comportant une zone de détection située sur le boîtier qui est délimitée par une discontinuité de surface telle qu'un creux ou un relief, **caractérisée en ce que** lesdits moyens de commande sont de type sensitif et en ce que le boîtier renferme un élément capteur associé à un circuit électronique de pilotage et un élément de mousse électrostatique dont une extrémité est appliquée contre l'élément capteur et son extrémité opposée est appliquée contre une zone de la face interne du boîtier disposée au droit de la zone de détection.

Préférentiellement, l'élément de mousse électrostatique, lorsqu'il sera en place entre la face interne du boîtier et l'élément capteur sera dans un état légèrement comprimé.

Par ailleurs dans une variante de mise en oeuvre de l'invention permettant de faciliter la mise en place du circuit support de l'élément capteur dans le boîtier, la partie de l'élément de mousse qui est en contact avec l'élément capteur aura une surface plus grande que celle de ce capteur et l'on donnera alors à la résistivité de cette partie une valeur qui sera supérieure à la résistivité de la partie centrale de l'élément de mousse. Par ailleurs la résistivité de la partie de l'élément de mousse en contact avec la face interne du boîtier pourra être inférieure à la résistivité de la partie centrale de l'élément de mousse. La résistivité de l'élément de mousse sera préférentiellement inférieure à 5 MΩ.cm.

On décrira ci-après, à titre d'exemple non limitatif, une forme d'exécution de la présente invention, en référence au dessin annexé sur lequel :
La figure 1 est une vue en perspective d'une caméra suivant l'invention.
La figure 2 est une vue partielle en coupe verticale et longitudinale de la caméra représentée sur la figure 1.
La figure 3 est une vue en coupe transversale de la caméra représentée sur la figure 1 suivant la ligne II-II de celle-ci.
La figure 4 est une vue partielle en coupe verticale et longitudinale d'une variante de mise en oeuvre d'une caméra suivant l'invention.

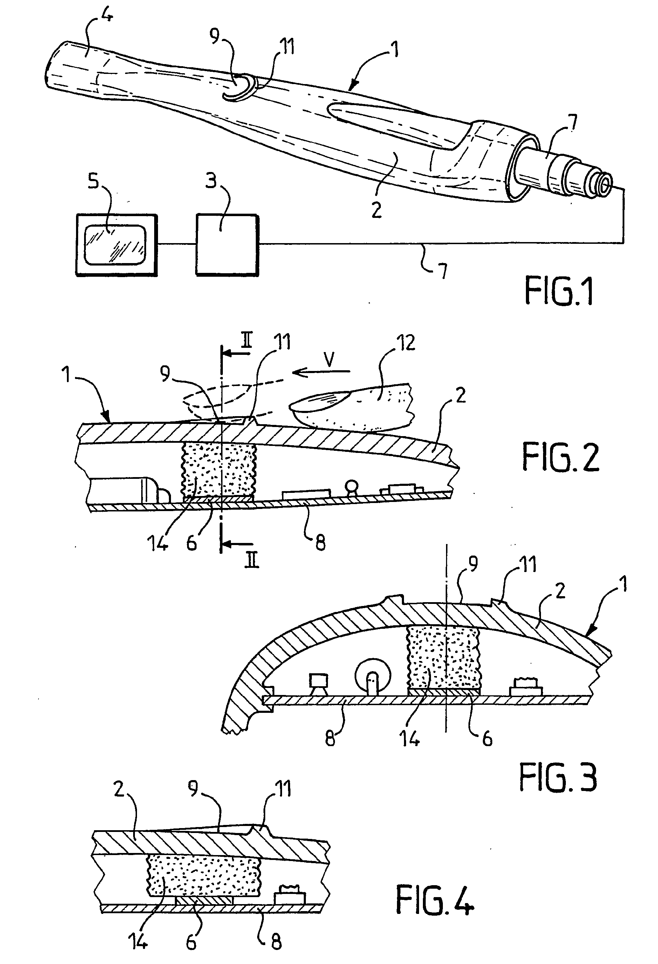

On a représenté sur les figures 1 à 3 une caméra dentaire 1 qui est constituée d'un corps allongé 2, de section droite sensiblement ellipsoïdale, comportant à son extrémité antérieure 4 un objectif de prise de vue et à son autre extrémité un cordon 7 par lequel elle est en liaison avec des moyens électroniques 3 d'alimentation et de gestion des images qui sont affichées sur un moniteur 5.

Cette caméra 1 comporte, suivant l'invention, un dispositif de commande permettant à l'utilisateur d'assurer le "gel" d'une image, c'est-à-dire de l'immobiliser sur le moniteur 5. Ce dispositif de commande comprend un capteur sensitif associé à un circuit électronique, et une zone de discontinuité de surface réalisée sur le boîtier 2.

La zone de discontinuité de surface est formée d'un bossage 11 en forme de C dont l'ouverture est orientée vers l'extrémité antérieure 4 de la caméra et qui fait légèrement saillie par rapport à la face supérieure du corps 2. Le bossage 11 est disposé en amont d'une zone 9, (dite zone de détection 9) par rapport au mouvement de déplacement naturel d'un doigt 12 de l'utilisateur lorsqu'il prend en main la caméra 1, à savoir le sens déterminé par la flèche V sur la figure 1.

On a disposé à l'intérieur du corps 2 un circuit imprimé 8, qui supporte un capteur sensitif, formé d'une pastille métallique 6, ainsi que les divers composants électroniques destiné d'une part à gérer la fonction de détection du capteur et d'autre part à commander la fonction à mettre en oeuvre consécutive à la détection, à savoir le "gel" de l'image.

Suivant l'invention, on propose de disposer entre le capteur 6 et la partie de la face interne du corps disposée au droit de la zone de détection 9, un élément de mousse 14 de type dit électrostatique, c'est-à-dire une mousse conductrice dont la résistivité est au moins égale à 5MΩ.cm. On a constaté que si une telle disposition permettait d'éviter les problèmes précédemment mentionnés en "déportant" en quelque sorte la zone de sensibilité du capteur 6 vers la paroi interne de la caméra située au droit de la zone de détection 9. Il a été établi que cette mousse devait être suffisamment conductrice pour propager l'effet capacitif du capteur 6 et suffisamment peu conductrice pour ne pas se comporter comme si elle constituait le capteur lui même.

On comprend que la présente invention est particulièrement intéressante pour les facilités qu'elle apporte en ce qui concerne la conception et la réalisation de ce type de caméra. En effet, d'une part, elle laisse au concepteur une liberté en ce qui concerne la forme du corps de la camera dans la mesure où le capteur peut être éloigné de la zone de détection, voire même décentré par rapport à celle-ci et d'autre part elle facilite l'assemblage.

Dans un mode de mise en oeuvre représenté sur la figure 4, on améliore la facilité de mise en place du circuit 8 et de l'élément de mousse 14 qui lui est associé en donnant à la base de celui-ci des dimensions supérieures à celles du capteur 6. Dans un tel mode de mise en oeuvre de l'invention, afin de minimiser les risques de courts-circuits susceptibles d'être générés par la face inférieure de la mousse lorsque celle-ci est amenée à venir en contact avec la face supérieure du circuit 8, on fera appel à un élément de mousse 14 dont la résistivité de la face inférieure sera plus élevée que celle de son centre.

Ainsi dans un exemple spécifique de mise en oeuvre de la présente invention, qui est représenté sur la figure 4, on fera appel à un capteur 6 de forme circulaire et de diamètre de l'ordre de 6 mm et à un élément de mousse de forme cylindrique de diamètre égal à 8 mm et de hauteur à l'état non comprimé de 5 mm, cet élément de mousse ayant après assemblage une épaisseur de 2,5mm. On a constaté qu'en choisissant une mousse électrostatique dont les résistivités des couches supérieure et inférieure étaient respectivement de 300 kΩ.cm et de 3000 kΩ.cm, la résistance entre les couches étant de 1500 kΩ.cm, la précision et la fiabilité de la détection étaient conservées par rapport à une disposition dans laquelle l'élément capteur 6 solidaire de son circuit 8 serait plaqué contre la face interne du corps de la caméra.

Dans ces conditions l'utilisation de la caméra suivant l'invention s'effectue ainsi qu'exposé ci-après. L'utilisateur, lorsque la caméra de prise de vue 1 est positionnée correctement, déplace l'un de ses doigts, par exemple le pouce 12 sur le corps 2 de celle-ci jusqu'à ce que ce dernier rencontre la butée 11. Dès lors, l'utilisateur sait que tout mouvement supplémentaire de son pouce dans la même direction V aura pour effet d'être détecté par le capteur sensitif 6 déclenchant ainsi l'opération souhaitée. On comprend que, dans la mesure où le déplacement du doigt de l'utilisateur s'effectue sur la surface du corps 2, en effleurant celui-ci et non perpendiculairement ainsi qu'il en serait dans le cas d'un interrupteur de type classique, ce mouvement ne peut provoquer de déviation brutale de la caméra 1.

## Revendications

1. Caméra de prise de vues médicale; notamment dentaire, comportant un boîtier allongé (2) apte à être tenu par une main d'un utilisateur et pourvu à son extrémité antérieure de moyens de prise d'images, ce boîtier (2) comportant des moyens de commande (6,8) aptes à "geler" sur des moyens d'affichage (5) une image choisie par l'utilisateur, ces moyens de commande comportant une zone de détection (9) située sur le boîtier (2) qui est délimitée par une discontinuité de surface telle qu'un creux ou un relief (11), **caractérisée en ce que** lesdits moyens de commande sont de type sensitif et **en ce que** le boîtier (2) renferme un élément capteur (6) associé à un circuit électronique (8) de pilotage et un élément de mousse électrostatique (14) dont une extrémité est appliquée contre l'élément capteur (6) et son extrémité opposée est appliquée contre une zone de la face interne du boîtier (2) disposée au droit de la zone de détection (9).

2. Caméra suivant la revendication 1, **caractérisée en ce que** l'élément de mousse électrostatique (14), lorsqu'il est en place entre la face interne du boîtier (2) et l'élément capteur (6) est dans un état légèrement comprimé.

3. Caméra suivant l'une des revendications 1 ou 2, **caractérisée en ce que** la partie de l'élément de mousse (14) en contact avec l'élément capteur (6) a une surface plus grande que celle de ce dernier.

4. Caméra suivant l'une des revendications précédentes, **caractérisée en ce que** la résistivité de la partie de l'élément de mousse (14) en contact avec l'élément capteur (6) est supérieure à la résistivité de la partie centrale de l'élément de mousse (14).

5. Caméra suivant l'une revendications précédentes, **caractérisée en ce que** la résistivité de la partie de l'élément de mousse (14) en contact avec la face interne du boîtier (2) est inférieure à la résistivité de la partie centrale de l'élément de mousse (14).

6. Caméra suivant l'une des revendications précédentes, **caractérisée en ce que** la résistivité de l'élément de mousse (14) est inférieure à 5 MΩ.cm.

7. Caméra suivant l'une des revendications précédentes, **caractérisée en ce que** l'épaisseur de l'élément de mousse (14) avant compression est de l'ordre de 5mm et la résistivité de la partie de celui-ci en contact avec la face interne du boîtier (2) est de l'ordre de 300 kΩ.cm, la résistivité de sa face opposée est de l'ordre de 3000 kΩ.cm et la résistivité de la partie centrale de l'élément de mousse (14) entre les couches extrêmes est de l'ordre de 1500 kΩ.cm.

## Claims

1. Camera for medical, particularly dental, use, comprising an elongated casing (2) adapted to be held in a user's hand and provided at its anterior end with image-taking means, this casing (2) comprising control means (6, 8) adapted to "freeze" on display means (5) an image chosen by the user, these control means comprising a zone of detection (9) located on the casing (2) which is defined by a surface discontinuity such as a hollow or a crest (11), **characterized in that** said control means are of sensitive type and **in that** the casing (2) contains a sensor element (6) associated with an electronic piloting circuit (8) and an electrostatic foam element (14) of which one end is applied against the sensor element (6) and its opposite end is applied against a zone of the inner face of the casing (2) disposed plumb with the zone of detection (9).

2. Camera according to claim 1, **characterized in that** the electrostatic foam element (14), when it is in position between the inner face of the casing (2) and the sensor element (6), is in a slightly compressed state.

3. Camera according to any one of claim 1 or 2, **characterized in that** the part of the foam element (14) in contact with the sensor element (6) has a larger surface than that of the latter.

4. Camera according to any one of the preceding claims, **characterized in that** the resistivity of that part of the foam element (14) in contact with the sensor element (6) is greater than the resistivity of the central part of the foam element (14).

5. Camera according to any one of the preceding claims, **characterized in that** the resistivity of that part of the foam element (14) in contact with the inner face of the casing (2) is less than the resistivity of the central part of the foam element (14).

6. Camera according to any one of preceding claims, **characterized in that** the resistivity of the foam element (14) is less than 5 MΩ.cm.

7. Camera according to any one of preceding claims, **characterized in that** the thickness of the foam element (14) before compression is of the order of 5 mm and the resistivity of that part thereof in contact with the inner face of the casing (2) is of the order of 300 KΩ.cm, the resistivity of its opposite face is of the order of 3000 kΩ.cm and the resistivity of the central part of the foam element (14) between the extreme layers is of the order of 1500 kΩ.cm.

## Patentansprüche

1. Medizinische, insbesondere zahnmedizinische Bildaufnahmekamera, mit einem länglichen Gehäuse (2), das geeignet ist, von einer Hand eines Benutzers gehalten zu werden und das an seinem vorderen Ende mit Bildaufnahmemitteln versehen ist, wobei dieses Gehäuse (2) Steuermittel (6, 8) aufweist, die geeignet sind, auf Anzeigemitteln (5) ein von dem Benutzer gewähltes Bild "einzufrieren", wobei diese Steuermittel einen an dem Gehäuse (2) befindlichen Erfassungsbereich (9) umfassen, der durch eine Oberflächenunterbrechung, wie eine Vertiefung oder eine Erhebung (11) begrenzt ist, **dadurch gekennzeichnet, daß** die Steuermittel sensitiver Art sind und daß das Gehäuse (2) ein einem elektronischen Steuerkreis (8) zugeordnetes Sensorelement (6) sowie ein elektrostatisches Schaumstoffelement (14) enthält, dessen eines Ende an dem Sensorelement (6) anliegt und dessen gegenüberliegendes Ende an einem direkt unter dem Erfassungsbereich (9) angeordneten Bereich der Innenseite des Gehäuses (2) anliegt.

2. Kamera nach Anspruch 1, **dadurch gekennzeichnet, daß** das elektrostatische Schaumstoffelement (14), wenn es zwischen der Innenseite des Gehäuses (2) und dem Sensorelement (6) angebracht ist, sich in einem leicht zusammengedrückten Zustand befindet.

3. Kamera nach einem der Ansprüche 1 oder 2, **dadurch gekennzeichnet, daß** der mit dem Sensorelement (6) in Kontakt befindliche Teil des Schaumstoffelements (14) eine größere Fläche als dieses Sensorelement aufweist.

4. Kamera nach einem der vorhergehenden Ansprüche, **dadurch gekennzeichnet, daß** der spezifische Widerstand des mit dem Sensorelement (6) in Kontakt befindlichen Teils des Schaumstoffelements (14) größer ist als der spezifische Widerstand des mittleren Teils des Schaumstoffelements (14).

5. Kamera nach einem der vorhergehenden Ansprüche, **dadurch gekennzeichnet, daß** der spezifische Widerstand des mit der Innenseite des Gehäuses (2) in Kontakt befindlichen Teils des Schaumstoffelements (14) geringer ist als der spezifische Widerstand des mittleren Teils des Schaumstoffelements (14).

6. Kamera nach einem der vorhergehenden Ansprüche, **dadurch gekennzeichnet, daß** der spezifische Widerstand des Schaumstoffelements (14) geringer als 5 MΩ.cm ist.

7. Kamera nach einem der vorhergehenden Ansprüche, **dadurch gekennzeichnet, daß** die Dicke des Schaumstoffelements (14) vor dem Zusammendrücken in der Größenordnung von 5 mm liegt und der spezifische Widerstand des mit der Innenseite des Gehäuses (2) in Kontakt befindlichen Teils dieses Elements in der Größenordnung von 300 kΩ.cm liegt, der spezifische Widerstand seiner gegenüberliegenden Seite in der Größenordnung von 3000 kΩ.cm und der spezifische Widerstand des mittleren Teils des Schaumstoffelements (14) zwischen den Außenschichten in der Größenordnung von 1500 kΩ.cm liegt.
